# EUROPEAN PATENT APPLICATION

(11) **EP 1 889 625 A1**
(43) Date of publication of application: **20.02.2008**
(21) Application number: 06117332.4
(22) Date of filing: 17.07.2006
(51) Int. Cl.: A61K 36/537, A61K 31/202, A61P 9/00

(54) **Dietary supplement for supporting mental and physical performance**

(71) Applicant: Pharmaton S.A., 6934 Bioggio (CH)
(72) Inventor: Camponovo, Fabrizio, 6832 Pedrinate (CH); Gianesello, Valter, 6945 Origlio (CH); Meixner, Wolfgang, 6949 Comano (CH); Vignutelli, Alberto, 6900 Lugano (CH)
(74) Representative: Eckhardt, Conrad

(57) **Abstract**

A dietary supplement is claimed, comprising at least one one ω-3 fatty acid and *Salvia lavadulaefolia* extract. The dietary supplement claimed herein provides support for mental and physical performance. Further, an oral dosage form is claimed, which comprises the dietary supplement. Moreover, a method for improving the mental and physical skills of a human by administering the oral dosage form or the dietary supplement is claimed.

## Description

### Field of the invention

The present invention relates to a dietary supplement containing at least one ω-3 fatty acid and *Salvia lavadulaefolia* extract. The dietary supplement claimed herein provides support for mental and physical performance.

### Background to the invention

It is widely known in the art that certain plant extracts are capable of delivering mental performance benefits to humans. Examples of such plant extracts are Ginseng extract, obtained from *Panax ginseng,* or Gingko extract, obtained from *Gingko biloba.* It is also known in the art that plant extracts like those mentioned above can be advantageously combined with further substances, such as vitamins, minerals, trace elements or fatty or amino acids. An exemplary reference of this kind is WO 2005/023229.

Although the above-mentioned compositions and mixtures have some advantageous effects on human health and well-being there is still a need to provide a dietary composition, which offers improved support for mental and physical performance.

### Summary of the invention

The present inventors have found that the above need can be addressed by providing a dietary supplement comprising:
a) at least one ω-3 fatty acid, and
b) *Salvia lavadulaefolia* extract.

Particularly, the present inventors have found that fish oil is a good quality and readily-available source for the ω-3 fatty acids used herein. From the latter, especially DHA (docosahexaenoic acid) and EPA (eicosapentaenoic acid) have been proven useful herein.

In another aspect of the present invention an oral dosage form is provided, which contains the dietary supplement of the invention.

In a further aspect of the invention a method for improving the mental and physical skills of a human by administering the oral dosage form is provided.

The present inventors have found that the combination of *Salvia lavadulaefolia* extract with ω-3 fatty acids, optionally in the presence of vitamins and minerals, aids maintaining the overall well-being and vitality of human beings by supporting memory and concentration, by maintaining the physical performance and helping to protect the cardiovascular system. These effects can be further promoted by the addition of vitamins and minerals.

### Detailed description of the invention

As a first key ingredient the dietary supplement of the present invention comprises *Salvia lavandulaefolia* extract. Preferably, *Salvia lavandulaefolia* extract is employed herein as *Salvia lavandulaefolia* essential oil. *"Salvia lavadulaefolia* essential oil" herein refers to substances or mixtures of substances, which are obtained from *Salvia lavadulaefolia* plants by conventional extraction techniques known to the person of ordinary skill in the art. The term *Salvia lavadulaefolia* extracts herein includes solids, oils, liquids, solutions, dispersions, emulsions and mixtures thereof. The composition of the essential oil of *Salvia lavadulaefolia* is listed in table 1. The *Salvia lavadulaefolia* essential oil can be obtained from Essencia Aetherische Oele AG, Stäffelistrasse 8, Winterthur, Switzerland. In typical embodiments herein the essential oil of *Salvia lavadulaefolia* is present as oil or oil supported on a solid matrix.

**Table 1**

| Content in % by weight | component |
|---|---|
| 1,00 | tricyclene |
| 0,30 | α-thujene |
| 10,00 | α-pinene |
| 5,00 | camphene |
| 1,50 | sabinene |
| 10,00 | β-pinene |
| 5,00 | myrcene |
| 0,20 | α-phellandrene |
| 0,5 | α-terpinene |
| 1,20 | para-cymene |
| 24,00 | 1,8-cineole |
| 5,00 | limonene |
| 0,70 | (Z)-β-ocimene |
| 1,00 | (E)-β-ocimene |
| 1,50 | γ-terpinene |
| 0,50 | trans-sabinene hydrate |
| 0,20 | terpinolene |
| 0,50 | linaool |
| 10,00 | camphor |
| 0,20 | δ-terpineol |
| 2,00 | borneol |
| 0,90 | terpinen-4-ol |
| 2,50 | α-terpineol |
| 0,10 | nerol |
| 0,25 | geraniol |
| 0,40 | linalyl acetate |
| 2,00 | bornyl acetate |
| 0,30 | cis-sabinyl acetate |
| 3,00 | α-terpinyl acetate |
| 0,20 | α-cubebene |
| 0,10 | geranyl acetate |
| 2,00 | β-caryophyllene |
| 0,30 | aromadendrene |
| 1,50 | α-humulene |
| 0,10 | allo-aromadendrene |
| 0,50 | bicyclogermacrene |
| 0,20 | δ-cadinene |
| 0,35 | spathulenol |
| 1,50 | caryophyllene oxide |
| 3,00 | viridiflorol |
| 0,10 | caryophyllenol |
| 0,10 | copaene |
| 0,10 | cis-alloocimene |
| | |
| 99,80% | 43 compounds |

As a second key ingredient the dietary supplement of the present invention comprises ω-3 fatty acids. Generally, the term "ω-3 fatty acids" or "omega-3 fatty acid" denominates organic fatty acids, which are provided with a C=C double bond in their chain at position 3 when counted from the methyl end of the acid molecule. In the context of the present invention DHA (4,7,10,13,16,19 - decosahexaenoic acid), EPA (5, 8, 11, 14, 17 - eicosapentaenoic acid), as free fatty acids, esters, glycerides are preferred. Particularly preferred ω-3 fatty acids herein are DHA and EPA. In a particular execution of the present invention the ratio of DHA to EPA ranges from 1:1 to 1:2 by weight, and is preferably 1:1.5 by weight. There are different sources of ω-3 fatty acids available. A readily available source of ω-3 fatty acids is fish oil. "Fish oil" as used herein refers to purified, winterised and deodorised fatty oil obtained from fish of the families *Engraulidae, Carangidae, Clupeidae, Osmeridae, Scombridae* and *Ammodytidae.* The ω-3 fatty acids are defined as the following acids: α-linolenic acid (C18:3 n-3), moroctic acid (C18:4 n-3), eicosatetraenoic acid (C20:4 n-3), timnodonic (eicosapentaenoic) acid (C20:5 n-3; EPA), heneicosapentaenoic acid (C21:5 n-3), clupanodonic acid (C22:5 n-3) and cervonic (docosahexaenoic) acid (C22:6 n-3; DHA). Fish oil is characterized by its content of EPA and DHA as well as by its total ω-3 fatty acids (all expressed as triglycerides), specifically fish oil contains at least 13% by weight of EPA, at least 9% by weight of DHA and at least 28% of total ω-3 fatty acids (cf. European Pharmacopoeia 5.05, O1/2005:1912, corrected 5.4). Fish oil can be obtained from CRODA GmbH, Herrenpfad-Sid 33, 41334 Nettetal, Germany under the trade name Incromega TG3322. An alternative source for ω-3 fatty acids is the oil derived from microalgae coming from families like Thraustochytriaceae. This microalgae oil has substantially the same composition as fish oil.

Optionally, the dietary supplement of the present invention comprises vitamins. Of the vitamins, both water-soluble and fat-soluble vitamins may be used. Suitable vitamins include water-soluble vitamins, such as vitamin C, e.g. L-(+)-ascorbic acid, calcium ascorbate, sodium ascorbate, potassium ascorbate, 6-palmitoyl-L-ascorbic acid; vitamin B1, e.g. thiamine hydrochloride, thiamine mononitrate; vitamin B2, e.g. riboflavin, riboflavin-5'-phosphate sodium; vitamin B6, e.g. pyridoxine hydrochloride, pyridoxine 5'-phosphate; vitamin B12, e.g. cyanocobalamine; vitamin H, e.g. D-biotin; folic acid; vitamin PP (niacin), e.g. nicotinamide, nicotinic acid; pro-vitamin B5, e.g. panthenol (D and DL forms), ethylpanthenol and calcium-D-pantothenate. Suitable fat-soluble vitamins are e.g. vitamin A, such as retinol, retinyl acetate, retinyl palmitate; vitamin D, e.g. ergocalciferol, cholecalciferol; vitamin E, e.g. the D and DL forms of alpha-tocopherol, alpha-tocopherylacetate, alpha-tocopherylic acid succinate; vitamin K, such as vitamin K1, e.g. phytomenadione, and carotenoids (provitamin), e.g. lycopene, zeaxanthine, lutein, alpha-carotene, beta-carotene, apocarotinal, gamma-carotene and beta-cryptoxanthine. Riboflavin is preferably used in salt form, e.g. as riboflavin phosphate, as the salt form has greater solubility). Preferred vitamins herein are selected from the group consisting of vitamins E, C, D, thiamine (B1), ribolflavin (B2), pyridoxine (B6), cobalamine (B12), folate, and mixtures thereof.

Further optionally, the dietary supplement of the present invention comprises minerals. Minerals herein refers to all minerals as listed in Annex II, chapter B of the Directive 2002/46/EC of the European Parliament and of the Council of June 10, 2002. Preferred minerals herein are minerals based on zinc, copper, iron, selenium and mixtures thereof. Preferred zinc minerals herein are zinc acetate, zinc chloride, zinc gluconate, zinc lactate, zinc oxide and zinc sulphate. Preferred copper minerals herein are copper lysine complex, cupric gluconate, cupric sulphate, cupric carbonate, cupric citrate. Preferred iron minerals herein are ferrous carbonate, ferrous citrate, ferric ammonium citrate, ferrous gluconate, ferrous fumarate, ferric sodium diphosphate, ferrous lactate, ferrous sulphate, ferric diphosphate, ferric saccharate, and elemental iron. Preferred selenium minerals herein are , such as sodium selenate, sodium selenite, sodium hydrogen selenite. Mixtures of the minerals mentioned herein are expressly also within the preferred scope of the present invention.

In a second aspect of the present invention an oral dosage form is provided, which comprises the dietary supplement described herein before. The oral dosage form herein is preferably a tablet or capsule. The capsule can be a soft or hard capsule with gelatine capsules being preferred. The tablet or gelatine capsule is produced by processes and from materials, which are known to the skilled person for such purposes. Further, the oral dosage form herein can be a powder or granules, which are to be administered orally. Alternatively, the oral dosage form can be a powder, which is to be dissolved in water prior to oral administration. Further examples for the oral dosage form herein are solutions or dispersions in solvents or dispersing agents suitable therefore.

In a preferred embodiment according to the invention the dosage form contains the aforementioned extract *of Salvia lavandulaefolia* in an amount ranging from 1 to 500µl, preferably from 20 to 80 µl, more preferably from 30 to 70 µl per dosage form. Of particular relevance according to the invention are dosage forms containing the aforementioned extract of *Salvia lavandulaefolia* in an amount ranging from 40 to 60 µl, more preferably from 45 to 55 µl per dosage form. The total amount of the ω-3 fatty acids mentioned hereinbefore is preferably ranging from 10 to 1000 mg, preferably from 20 to 300, more preferably from 30 to 200 mg and most preferably from 50 to 180 mg per dosage form.

As stated above, the supportive effects of the dietary supplement described herein on well being and vitality can be further promoted by adding vitamins and minerals. Thus, embodiments of the oral dosage form according to the present invention can be formed by adding any of the following compounds:
- from 0.1 to 100 mg of vitamin E;
- from 10 to 200 mg of vitamin C;
- from 0.05 to 50 µg of vitamin D;
- from 20 to 2000 µg of folate;
- from 0.005 to 20 mg of thiamine;
- from 0.005 to 20 mg of riboflavin;
- from 0.1 to 50 mg of pyridoxine;
- from 0.005 to 20 µg of cobalamin;
- from 0.1 to 100 mg of zinc;
- from 0.01 to 10 mg of copper(II);
- from 0.1 to 100 mg of iron;
- from 1 to 1000 µg of selenium.

In a preferred embodiment of the oral dosage form according to the present invention the amount of vitamin E ranges from 1 to 30 mg, preferably from 3 to 20 mg, more preferably from 5 to 15 mg and most preferably from 7 to 13 mg per dosage form.

In another preferred embodiment of the oral dosage form according to the present invention the amount of vitamin C ranges from 30 to 180 mg, preferably from 60 to 150 mg, more preferably from 70 to 130 mg and most preferably 80 - 100 mg per oral dosage form.

In yet another preferred embodiment of the oral dosage form according to the present invention the amount of vitamin D ranges from 0.1 to 20 µg, preferably from 0.5 to 12 µg, more preferably from 1 to 10 µg and most preferably from 3 to 7 µg per oral dosage form.

In yet another preferred embodiment of the oral dosage form according to the present invention the amount of folate ranges from 10 µg to 1000 µg, preferably from 50 to 600 µg, more preferably from 100 to 300 µg and most preferably from 150 to 250 µg per oral dosage form.

In yet another preferred embodiment of the oral dosage form according to the present invention the amount of thiamine (vitamin B1) ranges from 0.1 to 10 mg, preferably from 0.3 to 8 mg, more preferably from 0.5 to 5 mg and most preferably from 1 to 2 mg per oral dosage form.

In yet another preferred embodiment of the oral dosage form according to the present invention the amount of riboflavin (vitamin B2) ranges from 0.1 to 10 mg, preferably from 0.3 to 8 mg, more preferably from 0.5 to 5 mg and most preferably from 1 to 2 mg per oral dosage form.

In yet another preferred embodiment of the oral dosage form according to the present invention the amount of pyridoxine (vitamin B6) ranges from 0.2 to 20 mg, preferably from 0.5 to 12 mg, more preferably from 1 to 8 mg and most preferably from 1.5 to 2.5 mg per oral dosage form.

In yet another preferred embodiment of the oral dosage form according to the present invention the amount of cobalamine (vitamin B 12) ranges from 0.1 to 10 µg, preferably from 0.3 to 7 µg, more preferably from 0.5 to 5 µg and most preferably from 0.8 to 1.2 µg per oral dosage form.

In yet another preferred embodiment of the oral dosage form according to the present invention the amount of zinc ranges from 0.5 to 50 mg, preferably from 0.7 to 20 mg, more preferably from 1 to 10 mg and most preferably from 3 to 7 mg per oral dosage form.

In yet another preferred embodiment of the oral dosage form according to the present invention the amount of copper ranges from 0.05 to 5 mg, preferably from 0.07 to 2 mg, more preferably from 0.1 to 1 mg and most preferably from 0.3 to 0.7 mg per oral dosage form.

In yet another preferred embodiment of the oral dosage form according to the present invention the amount of iron ranges from 0.5 to 50 mg, preferably from 0.7 to 20 mg, more preferably from 1 to 10 mg and most preferably from 3 to 7 mg per oral dosage form.

In yet another preferred embodiment of the oral dosage form according to the present invention the amount of selenium ranges from 5 to 500 µg, preferably from 7 to 200 µg, more preferably from 10 to 100 µg and most preferably from 30 to 70 µg per oral dosage form.

In another aspect the present invention relates to a method of improving the mental and physical skills of a human by administering the oral dosage form described herein before. It has proven that by such administration overall well being and vitality can be significantly improved with humans. The main benefit areas are support for memory and concentration, maintaining of the physical performance and protection of the cardiovascular system.

### Examples

### Example 1

The following example illustrates a typical oral dosage composition according to the present invention, in particular a gelatine capsule containing the following composition of ingredients:

| | |
|---|---|
| *Salvia lavandulaefolia* extract. | 50 µl |
| Fish oil (containing 20 % DHA and 30 % EPA by weight) | 250 mg |
| Vitamin E | 10 mg |
| Vitamin C | 90 mg |
| Vitamin D | 5 µg |
| Folate | 200 µg |
| Thiamine | 1.4 mg |
| Riboflavin | 1.6 mg |
| Pyridoxine | 2 mg |
| Cobalamine | 1 µg |
| Zinc oxide | 6.22 mg |
| Cupric sulphate | 1.42 mg |
| Ferrous sulphate | 15.46 mg |
| Sodium selenite | 110 µg |

### Example 2

An alternative example for the composition of ingredients in a gelatine capsule according to the present invention is listed in the following table:

| | |
|---|---|
| *Salvia lavandulaefolia* extract. | 30 µl |
| Fish oil (containing 20 % DHA and 30 % EPA by weight) | 200 mg |
| Vitamin E | 7 mg |
| Vitamin C | 50 mg |
| Vitamin D | 3 µg |
| Folate | 150 µg |
| Thiamine | 1 mg |
| Riboflavin | 1.1 mg |
| Pyridoxine | 1.5 mg |
| Cobalamine | 0.7 µg |
| Zinc oxide | 4.7 mg |
| Cupric sulphate | 1.1 mg |
| Ferrous sulphate | 11.6 mg |
| Sodium selenite | 82.5 µg |

### Example 3

Another alternative example for the composition of ingredients in a gelatine capsule according to the present invention is listed in the following table:

| | |
|---|---|
| *Salvia lavandulaefolia* extract. | 70 µl |
| Fish oil (containing 20 % DHA and 30 % EPA by weight) | 300 mg |
| Vitamin E | 13 mg |
| Vitamin C | 130 mg |
| Vitamin D | 7 µg |
| Folate | 250 µg |
| Thiamine | 1.8 mg |
| Riboflavin | 2.1 mg |
| Pyridoxine | 2.5 mg |
| Cobalamine | 1.3 µg |
| Zinc oxide | 7.8 mg |
| Cupric sulphate | 1.8 mg |
| Ferrous sulphate | 19.3 mg |
| Sodium selenite | 137 µg |

## Claims

1. Dietary supplement for supporting mental and physical performance, the supplement comprising:
a) at least one ω-3 fatty acid, and
b) *Salvia lavandulaefolia* extract.

2. The dietary supplement of claim 1, wherein the ω-3 fatty acid is provided by fish oil.

3. The dietary supplement of any of the previous claims, wherein the one ω-3 fatty acid is selected from DHA, EPA and mixtures thereof.

4. The dietary supplement of claim 3, wherein the ratio of DHA to EPA ranges from 1:1 to 1:2 by weight..

5. The dietary supplement of claim 4, wherein the ratio of DHA to EPA is 1:1.5 by weight.

6. The dietary supplement of any of the previous claims, wherein the *Salvia lavandulaefolia* extract is *Salvia lavandulaefolia* essential oil.

7. The dietary supplement of any of the previous claims, further comprising vitamins.

8. The dietary supplement of claim 7, wherein the vitamins are selected from vitamin E, vitamin C, vitamin D, folate, thiamine, riboflavin, pyridoxine, cobalamin and mixtures thereof.

9. The dietary supplement of any of the previous claims, further comprising minerals.

10. The dietary supplement of claim 9, wherein the minerals are selected from zinc compounds, copper compounds, iron compounds, selenium compounds and mixtures thereof.

11. The dietary supplement of any of claims 9 or 10, wherein the zinc compounds are selected from zinc acetate, zinc chloride, zinc gluconate, zinc lactate, zinc oxide and zinc sulphate and mixtures thereof.

12. The dietary supplement of any of claims 9-11, wherein the copper compounds are selected from copper lysine complex, cupric gluconate, cupric sulphate, cupric carbonate, cupric citrate and mixtures thereof.

13. The dietary supplement of any of claims 9-12, wherein the iron compounds are selected from ferrous carbonate, ferrous citrate, ferric ammonium citrate, ferrous gluconate, ferrous fumarate, ferric sodium diphosphate, ferrous lactate, ferrous sulphate, ferric diphosphate, ferric saccharate, and elemental iron and mixtures thereof

14. The dietary supplement of any of claims 9-13, wherein the selenium compounds are selected from sodium selenate, sodium selenite, sodium hydrogen selenite and mixtures thereof.

15. An oral dosage form, comprising the dietary supplement of any of the previous claims.

16. The oral dosage form of claim 15, comprising:
i) from 1 to 500µl, preferably from 20 to 80 µl, more preferably from 30 to 70 µl, even more preferably from 40 to 60 µl and most preferably from 45 to 55 µl of *Salvia lavandulaefolia* essential oil.
ii) from 10 to 1000 mg, preferably from 20 to 300, more preferably from 30 to 200 mg and most preferably from 50 to 180 mg of a ω-3 fatty acid.

17. The oral dosage form of claim 16, further comprising any of the following compounds:
iii) vitamin E in an amount ranging from 1 to 30 mg, preferably from 3 to 20 mg, more preferably from 5 to 15 mg and most preferably 7 - 13 mg;
iv) vitamin C ranges from 30 to 180 mg, preferably from 60 to 150 mg, more preferably from 70 to 130 mg and most preferably 80-100 mg;
v) vitamin D ranges from 0.1 to 20 µg, preferably from 0.5 to 12 µg, more preferably from 1 to 10 µg and most preferably from 3 to 7 µg;
vi) folate ranges from 10 µg to 1000 µg, preferably from 50 to 600 µg, more preferably from 100 to 300 µg and most preferably from 150 to 250 µg;
vii) thiamine (vitamin B1) ranges from 0.1 to 10 mg, preferably from 0.3 to 8 mg, more preferably from 0.5 to 5 mg and most preferably from 1 to 2 mg;
viii) riboflavin (vitamin B2) ranges from 0.1 to 10 mg, preferably from 0.3 to 8 mg, more preferably from 0.5 to 5 mg and most preferably from 1 to 2 mg;
ix) pyridoxine (vitamin B6) ranges from 0.2 to 20 mg, preferably from 0.5 to 12 mg, more preferably from 1 to 8 mg and most preferably from 1.5 to 2.5 mg;
x) cobalamine (vitamin B12) ranges from 0.1 to 10 µg, preferably from 0.3 to 7 µg, more preferably from 0.5 to 5 µg and most preferably from 0.8 to 1.2 µg;
xi) zinc ranges from 0.5 to 50 mg, preferably from 0.7 to 20 mg, more preferably from 1 to 10 mg and most preferably from 3 to 7 mg;
xii) copper ranges from 0.05 to 5 mg, preferably from 0.07 to 2 mg, more preferably from 0.1 to 1 mg and most preferably from 0.3 to 0.7 mg;
xiii) iron ranges from 0.5 to 50 mg, preferably from 0.7 to 20 mg, more preferably from 1 to 10 mg and most preferably from 3 to 7 mg;
xiv) selenium ranges from 5 to 500 µg, preferably from 7 to 200 µg, more preferably from 10 to 100 µg and most preferably from 30 to 70 µg.

18. The oral dosage form of any of claims 15-17, comprising:
i) 50 µl *of Salvia lavandulaefolia* extract;
ii) 250 mg fish oil containing 20 % DHA and 30% EPA, by weight;
iii) 10 mg of vitamin E;
iv) 90 mg of vitamin C;
v) 5 µg of vitamin D;
vi) 200 µg of folate;
vii) 1.4 mg of thiamine;
viii) 1.6 mg of riboflavine;
ix) 2 mg of pyridoxine;
x) 1 µg of cobalamin;
xi) 5 mg of zinc;
xii) 0.5 mg of copper(II);
xiii) 5 mg of iron;
xiv) 50 µg of selenium.

19. The oral dosage forms of any of claims 15-18, wherein the oral dosage form is a tablet or a gelatine capsule.

20. A method for improving the mental and physical skills of a human by administering the oral dosage form of any of claims 15-19 or the dietary supplement of any of claims 1-14.
